# FASCICULE DE BREVET EUROPEEN

(11) **EP 3 703 105 B1**
(45) Date de publication et mention de la délivrance du brevet: **09.02.2022**
(21) Numéro de dépôt: 20159988.3
(22) Date de dépôt: 28.02.2020
(51) Int. Cl.: H01J 61/54, H05B 41/30, H01J 61/90

(54) **APPAREIL DE GÉNÉRATION DE SPECTRE UV OPTIMISÉ**
GERÄT ZUR ERZEUGUNG EINES OPTIMIERTEN UV-SPEKTRUMS
DEVICE FOR GENERATING OPTIMISED UV SPECTRUM

(30) Priorité: 28.02.2019 FR 1902113
(43) Date de publication de la demande: 02.09.2020
(73) Titulaire: Sanodev, 87069 Limoges (FR)
(72) Inventeur: PICARD, Nicolas, 87270 BONNAC LA COTE (FR); SANDOVAL, Laure, 87000 LIMOGES (FR); LAMARDELLE, Arnaud, 87270 CHAPTELAT (FR); OSTERMEYER, Hugo, 23000 LA CHAPELLE TAILLEFERT (FR); AGIER, Yaneck, 87000 LIMOGES (FR)
(74) Mandataire: Plasseraud IP

(56) Documents cités:
- JP-A- 2007 109 507
- JP-A- 2008 158 405
- US-A- 2 728 877
- US-A- 3 022 444
- US-A1- 2005 168 149
- Oda et al.: "Electrode stabilized Xe filled Flash Lamp as a DUV and VUV Point Source", , 31 octobre 2008 (2008-10-31), XP055658389, Extrait de l'Internet: URL:https://www.ushio.co.jp/en/technology/ lightedge/200810/100365.html [extrait le 2020-01-15]

## Description

### Arrière-plan de l'invention

La présente invention concerne la neutralisation de pathogènes en particulier dans les systèmes de désinfections pour les domaines de la santé, de l'agroalimentaire, de la viticulture pour ne citer que quelques domaines applicatifs.

Les techniques utilisées principalement repose sur des techniques de lavage, de chauffage, d'utilisation d'ultra-sons et d'expositions aux rayonnements hyperfréquences et ultra-violet (UV).

Plus particulièrement, l'invention concerne les systèmes de désinfection utilisant la lumière pulsée dans le domaine de l'ultra-violet. En effet, des études scientifiques ont pu identifier les effets bactéricides du rayonnement de la lumière pulsée dans certaines longueurs d'onde du spectre électromagnétique. Les longueurs d'ondes spécifiques responsables de ces effets sont celles situées entre 100 et 300 nanomètres (nm), avec un pic à 254 nm. Ces longueurs d'onde sont situées dans le domaine des UV-C.

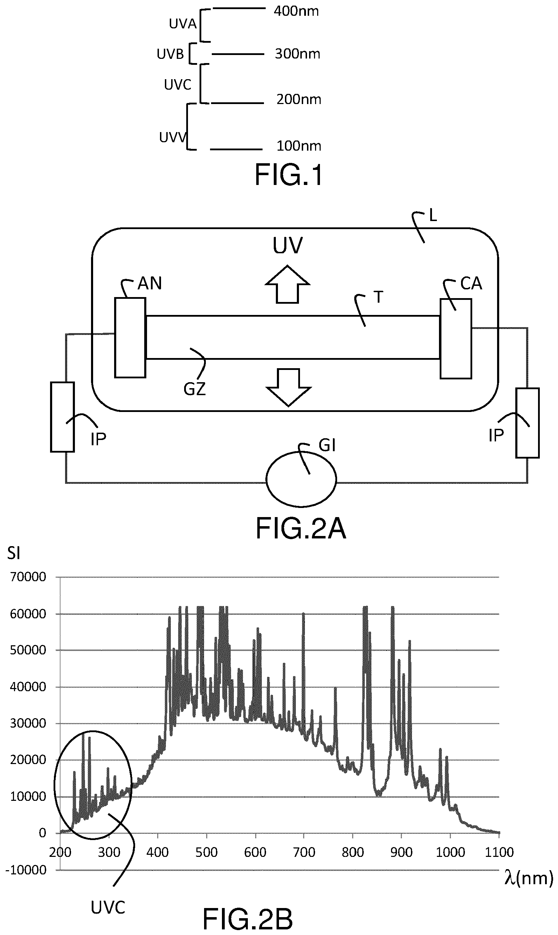

La figure 1 montre le domaine des radiations électromagnétiques dans le domaine des ultra-violet UV selon les longueurs d'onde en nanomètres. Ce domaine comprend quatre sous-domaines, les UVA correspondant à la lumière noire entre 315 et 400 nm, les UVB correspondant aux UV solaires entre 280 et 315 nm, les UVC correspondant aux longueurs d'onde germicides qui intéressent l'invention entre 200 et 280 nm avec un pic à 250 nm pour l'effet germicide et enfin les UV-vide UVV entre 100 et 200 nm.

Les sources de lumière pulsée sont généralement des tubes contenant un gaz spécifique dans lequel on provoque une décharge. Une impulsion haute tension est utilisée pour générer un streamer, une sorte de mini-arc dans le gaz. Puis une fois le canal ouvert, on libère une seconde impulsion avec une grande quantité d'énergie électrique pour exciter le milieu et générer un large spectre lumineux.

Les paramètres électriques de l'impulsion très énergétique sont déterminants dans la répartition spectrale du rayonnement. Tout l'enjeu consiste à maximiser le rayonnement dans le domaine de l'UV-C. Certaines bactéries sont alors très sensibles à ce type de rayonnement conduisant à leurs destructions.

L'état de l'art comprend de nombreuses publications sur de telles sources de lumière puisée. En particulier, la figure 2A montre un schéma de lampe UV L avec un générateur d'impulsion haute tension GI et sa connectique. La lampe L comprend une anode AN et une cathode CAT ainsi qu'un tube T comprenant un gaz GZ adéquat. L'émission UV générée par le tube T est schématisée par des flèches. Une impulsion d'une amplitude d'au moins 3 kV produite dans le montage électrique connecté entre l'anode AN et la cathode CAT permet la création d'un arc électrique entre anode AN et cathode CAT. Typiquement, le générateur d'impulsion est apte à produire des impulsions d'une durée de 20 à 40 µs avec un temps de montée d'une durée inférieure à 3 µs. De telles caractéristiques d'impulsion sont adaptées à la production d'un rayonnement ultra-violet adapté aux besoins de l'invention.

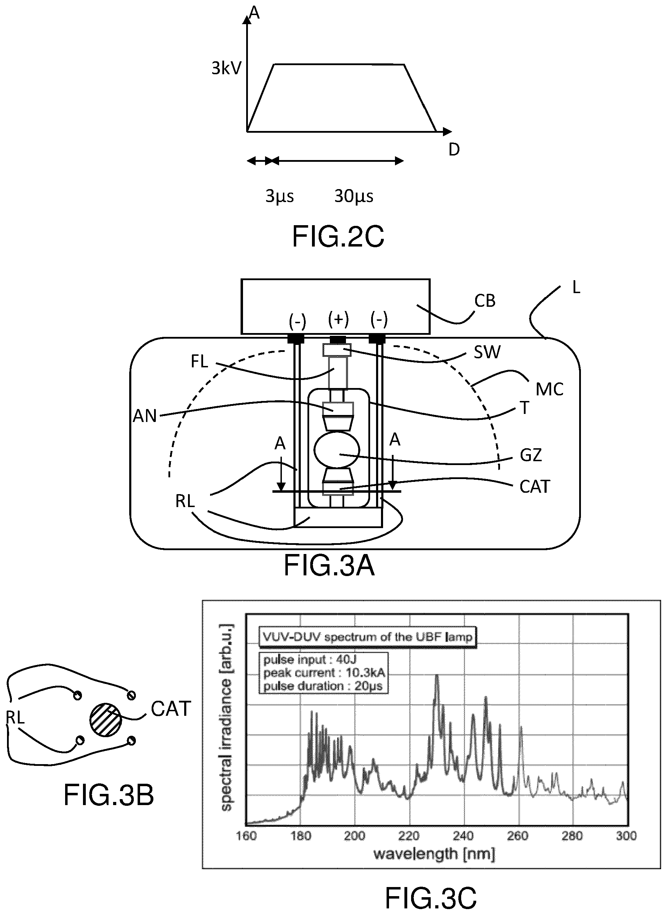

L'impulsion générée va donc exciter le gaz GZ de la lampe L et émettre un large spectre comme celui présenté sur la figure 2B. Plus l'impulsion est brève avec un temps de montée très court, plus le spectre émis sera riche en UV-C. Typiquement l'impulsion a une amplitude de l'ordre de quelques kV (ou de l'ordre de 10kA) pendant 20 à 40µs avec un temps de montée de l'ordre de quelques µs. Un exemple est schématiquement représenté sur la figure 2C montrant l'amplitude A en fonction de la durée D. Typiquement une amplitude de 3 kV est adéquate avec une montée d'une durée de 3 µs, l'impulsion durant 30 µs avant de redescendre en 3 µs.

La connectique a une incidence sur la forme de l'impulsion excitant réellement le gaz qui est ainsi fortement modifiée et déformée ce qui réduit son efficacité. En effet les connexions, les longueurs de câbles et la géométrie présentent une inductance parasite IP.

On voit sur la figure 2B qu'il y a peu d'énergie dans les UV-C, environ 10% seulement.

Afin de palier à cet inconvénient, une solution de connexion coaxiale est proposée dans le document Electrode stabilized Xe filled Flash Lamp as a DUV and VUV Point Source, de F.Oda, M. Ikeuchi, Y. Morimoto, Ushio Inc., 1194, Sazuchi, Bessho-cho, Himeji. Hyogo 671-0224.

Les figures 3A et 3B montrent la solution proposée dans ce document et la figure 3C montre le spectre ainsi obtenu. L'architecture coaxiale permet de réduire fortement l'impact inductif de la connexion entre une banque de condensateur CB et la lampe L et de ce fait de moins déformer et étaler l'impulsion qui arrive effectivement à la lampe L.

Cette solution propose une géométrie particulière de la connectique et de la géométrie de la lampe L. Celle-ci présente une banque de condensateurs CB comprenant une borne positive + et une borne négative - auxquelles est connecté un générateur haute tension non représenté. La borne positive + est reliée à l'anode AN de la lampe L par une ligne d'alimentation FL et la borne négative - est reliée à la cathode CAT de la lampe L par une ligne de retour RL. La ligne d'alimentation FL est connectée à la banque de condensateurs CB par l'intermédiaire d'un interrupteur SW, qui peut être un thyristor ou un transistor. L'anode AN et la cathode CAT baigne dans un plasma GZ. C'est ici l'actionnement du commutateur SW qui permet la génération de l'impulsion par connexion de la banque de condensateurs CB chargée par le générateur haute tension. Un miroir de concentration du rayonnement MC complète la lampe L.

Dans une première phase, la banque de condensateurs CB se charge à la tension nominale. Dans une seconde phase, un ordre de déclenchement est envoyé sur le switch SW. Dans une troisième phase, la lampe L étant dimensionnée pour cela et donc étant relativement petite, le gaz GZ est ionisé uniquement par la tension présente dans la banque de condensateurs CB.

La lampe L fonctionne par exemple avec une tension de 600 à 1500Volts. La valeur capacitive de la banque de condensateurs CB se situe entre 80 et 250 µF.

La figure 3C montre l'amplitude spectrale obtenue dans le domaine des UVC en fonction de la longueur d'onde pour une impulsion de 40 joules, un courant de pic de 10.3 kA et une durée d'impulsion de 20 µs.

On remarque que le spectre est assez dispersé entre les longueurs d'onde de 180nm à 260nm.

La figure 3B montre une vue en coupe transversale AA telle que montrée sur la figure 3A, cette coupe montrant l'aspect coaxial de la structure. On y voit que la cathode CAT est située au centre des conducteurs RL auxquels elle est reliée, ces conducteurs radiaux RL permettant de remonter le courant vers la borne négative de la banque de condensateurs CB.

Les solutions conventionnelles dispersent l'énergie dans des longueurs d'onde de 100nm à 1300 nm avec une faible énergie dans l'UV-C. Or pour maximiser l'effet germicide il faut générer un spectre très riche en UV-C en particulier entre 100 nm et 300 nm.

On voit sur la figure 3C que, dans l'état de l'art, l'énergie disponible en UVC reste en dessous de 10%.

L'invention se propose de résoudre cette limitation en décrivant un système électronique qui optimise et maximise le rayonnement UVC afin d'augmenter son effet bactéricide.

### Exposé de l'invention :

La présente invention a donc pour but principal de pallier les défauts des solutions de l'art antérieur en proposant un dispositif de désinfection utilisant une lumière pulsée dans le domaine de l'ultra-violet comprenant au moins une chambre de désinfection, un générateur haute tension, une lampe ultra-violet comprenant une anode et une cathode placée dans un tube contenant un gaz spécifique apte à produire un rayonnement ultra-violet par excitation du gaz, un dispositif de déclenchement d'une impulsion entre anode et cathode et un montage coaxial d'alimentation de la lampe ultra-violet disposé entre le générateur d'impulsion et la lampe ultra-violet, ce montage coaxial comprenant une ligne d'alimentation reliée à l'anode et une ligne de retour reliée à la cathode, caractérisé en ce qu'un réseau de condensateurs est distribué le long du montage coaxial entre la ligne d'alimentation et la ligne de retour, ce réseau de condensateurs participant à la génération de l'impulsion par production de tension électrique entre l'anode et la cathode de la lampe en combinaison avec le dispositif de déclenchement, les caractéristiques du réseau de condensateurs étant optimisées pour améliorer la densité surfacique de courant fournie à la lampe et ainsi réduire la dispersion du spectre ultra-violet dans les longueurs d'onde en dehors des longueurs d'onde du domaine des ultra-violets C possédant des propriétés germicides.

L'invention consiste ainsi à distribuer un réseau de condensateurs le long du montage coaxial de l'art antérieur afin d'améliorer la densité surfacique de courant. Lors du passage de l'impulsion, une répartition homogène a lieu dans les conducteurs et permet à l'onde de se propager avec une très faible déformation. La répartition de condensateurs élémentaires le long de la connexion entre le générateur et la lampe permet de réduire les pertes en ligne inductives et résistives. Ces pertes étant responsables de la déformation de l'impulsion et, par conséquent, de l'atténuation et de la dispersion dans l'ensemble du spectre.

Avantageusement, le réseau de condensateurs est distribué sur une longueur d'au moins la longueur de la lampe.

Cette caractéristique, en combinaison avec la précédente, permet un dimensionnement optimal du dispositif de désinfection tout en assurant les propriétés de l'invention.

Le dispositif de déclenchement peut être un commutateur du type de l'art antérieur connecté à l'anode de la lampe en sortie de la borne positive du réseau de condensateurs du montage coaxial. Cependant l'effet d'un tel commutateur n'est pas neutre en termes de spectre d'énergie produite. On observe une dispersion diminuée par rapport aux solutions de l'art antérieur grâce à la distribution coaxiale particulière du réseau de condensateurs mais encore trop importante pour complètement optimiser l'énergie dans la bande spectrale utile pour la désinfection. Aussi, l'invention propose une diminution encore plus importante de la dispersion énergétique en proposant une caractéristique supplémentaire au dispositif de désinfection de l'invention.

Ainsi, selon une réalisation préférentielle, le dispositif de déclenchement comprend une bague de déclenchement disposée autour du tube pour y créer un champ ionisant et reliée pour cela à un générateur de déclenchement permettant d'ioniser le gaz présent dans le tube pour que la tension présente dans le réseau de condensateurs chargé par le générateur provoque une impulsion entre anode et cathode de la lampe.

Avec cette réalisation en combinaison avec une ou plusieurs des caractéristiques précédentes, le déclenchement de l'impulsion ne nécessite pas de commutateur entre la banque de condensateurs et la lampe comme dans le cas de l'art antérieur décrit sur la figure 3A. Ainsi le montage coaxial est dépourvu de commutateur directement connecté à une électrode de la lampe. Ici, le dispositif de déclenchement est extérieur à l'ensemble lampe-réseau de condensateur. Cela permet de réduire les pertes en ligne du signal arrivant sur l'anode AN et les effets dispersifs de la commutation. Cela permet en outre un échange facile de la lampe car la bague de déclenchement est externe, ce qui n'est généralement pas le cas lorsqu'un dispositif de déclenchement type commutateur est utilisé. En combinaison avec l'action ionisante de la bague de déclenchement alimentée par le générateur de déclenchement, le réseau de condensateur également chargé permet la génération d'un arc entre cathode et anode et une impulsion est ainsi générée dans le circuit produisant une émission d'énergie avec une concentration spectrale importante dans la zone considérée pour la désinfection et une dispersion énergétique minimale.

Selon un mode de réalisation avantageux, la répartition linéique des condensateurs est répartie d'une manière cylindrique.

Cela permet d'optimiser le caractère coaxial du montage électrique avec tous ses avantages en termes de résultat sur l'émission énergétique observée.

Selon une réalisation avantageuse, le générateur relié à la lampe par le montage coaxial est aussi le générateur de déclenchement.

Un tel générateur multi-sortie permet d'assurer une compacité au dispositif obtenu et un cout maitrisé pour la réalisation du dispositif.

Selon une implémentation avantageuse, la densité surfacique de courant est telle que l'énergie fournie à la lampe est comprise entre 20 et 500 Joules.

Avec cette amplitude d'énergie, on assure que le spectre en UVC sera suffisamment efficace pour l'application envisagée.

Une densité surfacique de courant telle que l'énergie fournie à la lampe est comprise entre 40 et 50 Joules est particulièrement intéressante car une puissance de 40 Joules est généralement suffisante pour détruire les bactéries considérées.

Selon une implémentation préférentielle, le réseau de condensateurs présente une capacité de C=2E/V² en fonction de l'énergie E optimale et de la tension pic V du signal généré.

Une telle capacité assure une maitrise de l'énergie fournie à la lampe ultra-violet.

Selon une optimisation avantageuse de l'invention, le gaz de la lampe ultra-violet est excité par une tension d'au moins 500 V entre l'anode et la cathode, générée par le réseau de condensateurs chargé par le générateur.

Une telle tension permet la génération de l'arc électrique pour des lampes de dimension classique pour ce genre d'application. Des dimensions plus importantes peuvent requérir une tension dépassant le millier de Volts, typiquement 3 Kv.

Selon une autre optimisation avantageuse de l'invention, le réseau de condensateur est apte à produire des impulsions d'une durée de 20 à 40 µs avec un temps de montée d'une durée inférieure à 3 µs en combinaison avec le dispositif de déclenchement.

Ces caractéristiques permettent d'assurer un bon comportement désinfectant du dispositif obtenu.

Dans un mode de réalisation avantageux, le réseau de condensateurs comprend une pluralité de composants capacitifs soudés entre la ligne d'alimentation et la ligne de retour au sein du montage coaxial.

Ce mode de réalisation permet de construire le réseau de condensateurs de manière optimale en contrôlant la conductance introduite entre ligne d'alimentation et ligne de retour.

L'invention concerne aussi un système de désinfection comprenant plusieurs dispositifs de désinfection selon l'invention en parallèle pour couvrir des surfaces à traiter d'autant plus étendues.

Cela permet d'obtenir une surface de désinfection d'autant plus grande que le nombre de dispositif est important.

Selon une réalisation avantageuse, un même générateur est connecté aux montages coaxiaux de plusieurs dispositifs selon l'invention.

Cela permet de mutualiser un même générateur pour une surface de traitement élargie II en est bien sûr de même pour ce qui concerne le générateur de déclenchement dans le cas d'une mutualisation également. De manière optimale, un seul et même générateur peut être utilisé pour la charge du réseau de condensateurs et le déclenchement par une bague de déclenchement.

Dans une application particulière, la mise en parallèle permet de traiter plusieurs processus.

Cela s'applique à une utilisation extensive du système pour diverses application, par exemple médicales et alimentaires.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1 montre les grands domaines du spectre électromagnétique connu comprenant le domaine des ultra-violets germicides UV-C ;
- les figures 2A, 2B, 2C montrent schématiquement respectivement une lampe UV et un générateur telle qu'utilisés dans un dispositif de désinfection de l'art antérieur, un spectre ainsi généré et un exemple d'impulsion utilisé pour produire un rayonnement adéquat dans le domaine électromagnétique recherché ;
- les figures 3A, 3B et 3C montrent une architecture coaxiale de l'art antérieur permettant de diminuer l'impact inductif du montage électrique entre le générateur et la lampe ultra-violet, schématiquement, une section transversale de cette structure coaxiale et le spectre obtenu avec l'architecture coaxiale de la figure 3A et 3B ;
- les figures 4A, 4B, 4C montrent schématiquement un montage électrique d'alimentation d'une lampe ultra-violet selon l'invention, en vue de côté et en coupe, et le spectre obtenu ;
- la figure 5 montre un dispositif de désinfection selon l'invention.

### Description détaillée de modes de réalisation

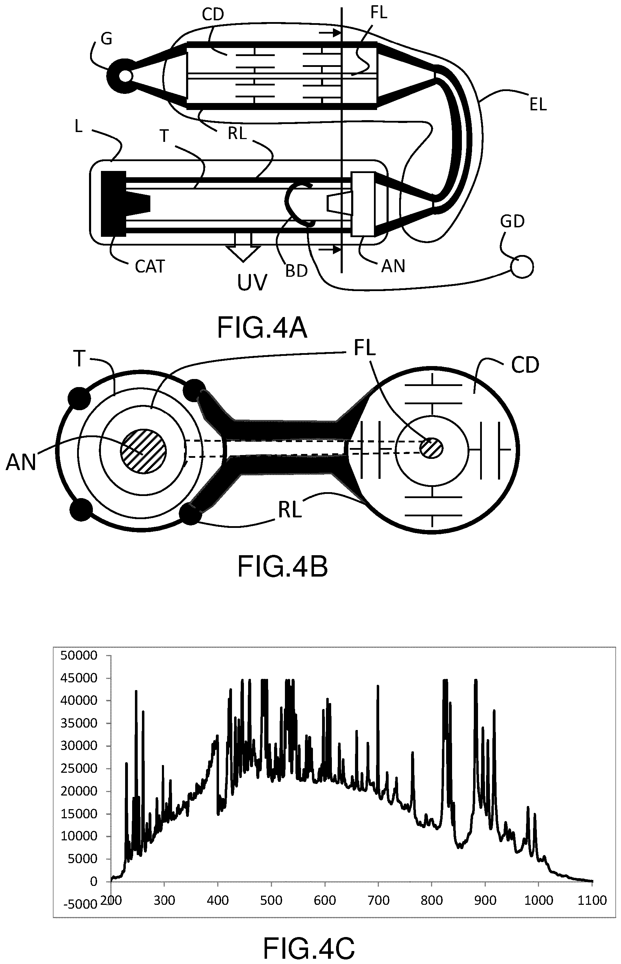

La figure 4A montre schématiquement un ensemble de composants essentiels à l'implémentation d'un dispositif de désinfection selon l'invention comprenant un générateur G, une lampe ultra-violet L comprenant un tube T, une anode AN et une cathode CAT et un montage électrique de l'invention comprenant un réseau de condensateurs CD distribués entre une ligne d'alimentation FL alimentant l'anode AN de la lampe L et une ligne de retour RL reliée à la cathode CAT. La figure 4B montre une vue en coupe enrichie de l'ensemble montré sur la figure 4A. Sur la figure 4B, sont ainsi représentés schématiquement les conducteurs de la ligne d'alimentation FL en hachurés, car intersectant le plan de coupe montré sur la figure 4A, et en pointillés, pour montrer schématiquement le caractère coaxial de la connexion sur tout son trajet. En fonction de l'application et de la quantité de rayonnement souhaitée, la longueur de lampe est avantageusement choisie pour dissiper cette énergie. Les lampes entre 5 et 50 cms permettront en particulier d'atteindre un rayonnement entre 20 et 500 Joules.

Avantageusement, la tension de charge des condensateurs est de 500 à 5000 volts et la valeur capacitive est de 10 à 400 µF. Le temps de décharge dans la lampe se situe de 2 à 80 µs avec une tension de déclenchement de 15 à 40 kV. La connexion du réseau de condensateur coaxial à l'anode de la lampe peut être réalisée par commutation.

Cependant, dans une réalisation préférentielle, une bague de déclenchement BD entourant le tube T de la lampe UV est utilisée pour ioniser le gaz dans le tube T par création d'un champ électrique ionisant dans le tube T. La pré-ionisation du gaz GZ par l'alimentation de la bague de déclenchement BD permet à la tension présente entre anode et cathode grâce au réseau de condensateurs chargé de finir par déclencher un arc électrique entre anode et cathode.

La valeur des condensateurs CD distribués se calcule selon l'énergie que l'on souhaite voir rayonner. On peut faire varier leurs capacités C entre quelques dizaines de µF jusqu'à quelques centaines de µF, préférentiellement de 10 à 400 µF.

Dans un mode de réalisation préférentiel, les condensateurs sont des composants soudés entre la ligne d'alimentation FL et la ligne de retour RL. Ces condensateurs sont soudés sur une partie de la longueur totale du montage coaxiale selon une répartition linéique de l'ordre de 7 à 12 µF/dm afin de limiter les effets inductifs et résistifs. L'ensemble est ajusté par la simulation de la charge et de la décharge du courant dans la banque de condensateur.

La figure 4C montre ainsi le spectre optimisé obtenu. On voit ainsi que l'énergie en UV-C est beaucoup plus importante que dans les cas précédents et focalisée autour des 250nm ce qui est l'effet recherché pour une efficacité bactéricide maximale.

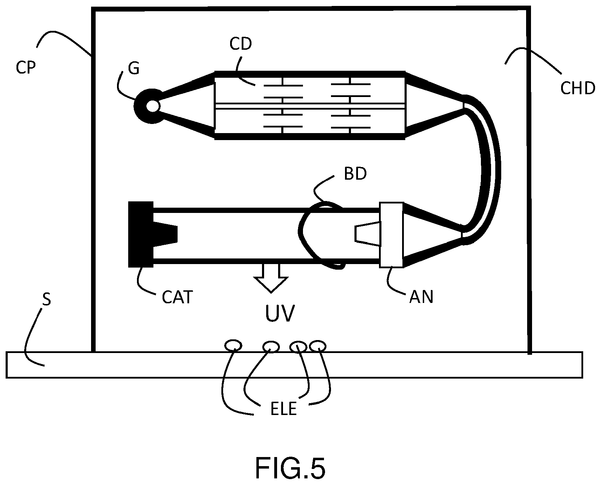

La figure 5 montre un dispositif de désinfection utilisant l'ensemble formé par le générateur, la lampe ultra-violet et le montage électrique de l'invention en combinaison avec une chambre de désinfection CHD, formée typiquement par une cage de protection CP et un support S, dans laquelle les éléments à désinfecter ELE ont été introduits sur le support S.

On remarque enfin que diverses mises en oeuvre peuvent être réalisées selon les principes de l'invention.

## Revendications

1. Dispositif de désinfection utilisant une lumière pulsée dans le domaine de l'ultra-violet comprenant au moins une chambre de désinfection (CHD), un générateur haute tension (GI), une lampe ultra-violet (L) comprenant une anode (AN) et une cathode (CAT) placée dans un tube (T) contenant un gaz spécifique (GZ) apte à produire un rayonnement ultra-violet par excitation du gaz (GZ), un dispositif de déclenchement d'une impulsion entre anode (AN) et cathode (CAT) et un montage coaxial (EL) d'alimentation de la lampe ultra-violet (L) disposé entre le générateur (GI) et la lampe ultra-violet (L), ce montage coaxial (EL) comprenant une ligne d'alimentation (FL) reliée à l'anode (AN) et une ligne de retour (RL) reliée à la cathode (CAT), **caractérisé en ce qu'**un réseau de condensateurs (CD) est distribué le long du montage coaxial (EL) entre la ligne d'alimentation (FL) et la ligne de retour (RL) sur une longueur d'au moins la longueur de la lampe (L), ce réseau de condensateurs participant à la génération de l'impulsion par production de tension électrique entre l'anode (AN) et la cathode (CAT) de la lampe (L) en combinaison avec le dispositif de déclenchement, pour lequel les caractéristiques du réseau de condensateurs (CD) sont optimisées pour améliorer la densité surfacique de courant fournie à la lampe (L) et ainsi réduire la dispersion du spectre ultra-violet dans les longueurs d'onde en dehors des longueurs d'onde du domaine des ultra-violets C possédant des propriétés germicides.

2. Dispositif de désinfection selon la revendication 1, **caractérisé en ce que** le réseau de condensateurs (CD) est distribué sur une longueur d'au moins la longueur de la lampe (L).

3. Dispositif de désinfection selon la revendication 1 ou 2, **caractérisé en ce que** le dispositif de déclenchement comprend une bague de déclenchement (BD) disposée autour du tube (T) pour y créer un champ ionisant et reliée pour cela à un générateur de déclenchement (GD) permettant d'ioniser le gaz (GZ) présent dans le tube (T) pour que la tension présente dans le réseau de condensateurs chargé par le générateur (G) provoque une impulsion entre anode et cathode de la lampe.

4. Dispositif de désinfection selon la revendication 1, 2 ou 3, **caractérisé en ce que** la répartition linéique des condensateurs du réseau de condensateurs est répartie d'une manière cylindrique.

5. Dispositif de désinfection selon l'une quelconque des revendications précédentes, **caractérisé en ce que** le générateur relié à la lampe par le montage coaxial est aussi le générateur de déclenchement.

6. Dispositif de désinfection selon l'une quelconque des revendications précédentes, **caractérisé en ce que** la densité surfacique de courant est telle que l'énergie fournie à la lampe est comprise entre 20 et 500 Joules.

7. Dispositif de désinfection selon la revendication 6, **caractérisé en ce que** la densité surfacique de courant optimisée est telle que l'énergie fournie à la lampe est comprise entre 40 et 50 Joules.

8. Dispositif de désinfection selon l'une quelconque des revendications précédentes, **caractérisé en ce que** le réseau de condensateurs (CD) présente une capacité de C=2E/V² en fonction de l'énergie E optimale et de la tension pic V du signal généré.

9. Dispositif de désinfection selon l'une quelconque des revendications précédentes, **caractérisé en ce que** le gaz (GZ) de la lampe ultra-violet (L) est excité par une tension d'au moins 500 V entre l'anode (AN) et la cathode (CAT), générée par le réseau de condensateurs chargé par le générateur.

10. Dispositif de désinfection selon l'une quelconque des revendications précédentes, **caractérisé en ce que** le réseau de condensateur (CD) est apte à produire des impulsions d'une durée de 20 à 40 µs avec un temps de montée d'une durée inférieure à 3 µs en combinaison avec le dispositif de déclenchement.

11. Dispositif de désinfection selon l'une quelconque des revendications précédentes, **caractérisé en ce que** le réseau de condensateurs (CD) comprend une pluralité de composants capacitifs soudés entre la ligne d'alimentation (FL) et la ligne de retour (RL) au sein du montage coaxial (EL).

12. Système de désinfection comprenant plusieurs dispositifs de désinfection selon l'une quelconque des revendications précédentes en parallèle pour couvrir des surfaces d'autant plus étendues.

13. Système de désinfection selon la revendication 12, **caractérisé en ce qu'**un même générateur est connecté aux montages coaxiaux de plusieurs dispositifs selon l'invention.

14. Système de désinfection selon la revendication 12, **caractérisée en ce que** la mise en parallèle desdits dispositifs permet de traiter plusieurs processus.

## Patentansprüche

1. Desinfektionsvorrichtung, die ein gepulstes Licht im ultravioletten Bereich verwendet, umfassend wenigstens eine Desinfektionskammer (CHD), einen Hochspannungsgenerator (GI), einer Ultraviolettlampe (L) mit einer Anode (AN) und einer Kathode (CAT), die in einem Rohr (T) angeordnet ist, das ein spezifisches Gas (GZ) enthält, das durch Anregung des Gases (GZ) eine ultraviolette Strahlung erzeugen kann, eine Vorrichtung zur Auslösung eines Impulses zwischen Anode (AN) und Kathode (CAT) und eine Koaxialanordnung (EL) zur Versorgung der Ultraviolettlampe (L), die zwischen dem Generator (GI) und der Ultraviolettlampe (L) angeordnet ist, wobei diese Koaxialanordnung (EL) eine mit der Anode (AN) verbundene Versorgungsleitung (FL) und eine mit der Kathode (CAT) verbundene Rückleitung (RL) umfasst, **dadurch gekennzeichnet, dass** ein Kondensatornetzwerk (CD) entlang der Koaxialanordnung (EL) zwischen der Versorgungsleitung (FL) und der Rückleitung (RL) über eine Länge von wenigstens der Länge der Lampe (L) verteilt ist, wobei dieses Kondensatornetzwerk an der Erzeugung des Impulses durch Erzeugung einer elektrischen Spannung zwischen der Anode (AN) und der Kathode (CAT) der Lampe (L) in Kombination mit der Auslösevorrichtung beteiligt ist, wobei die Eigenschaften des Kondensatornetzwerkes (CD) optimiert sind, um die der Lampe (L) verliehene Stromflächendichte zu verbessern und so die Streuung des ultravioletten Spektrums in Wellenlängen außerhalb der Wellenlängen des ultravioletten C-Bereichs mit keimtötenden Eigenschaften zu verringern.

2. Desinfektionsvorrichtung nach Anspruch 1, **dadurch gekennzeichnet, dass** das Kondensatornetzwerk (CD) über eine Länge von wenigstens der Länge der Lampe (L) verteilt ist.

3. Desinfektionsvorrichtung nach Anspruch 1 oder 2, **dadurch gekennzeichnet, dass** die Auslösevorrichtung einen Auslösering (BD) umfasst, der um die Röhre (T) herum angeordnet ist, um dort ein ionisierendes Feld zu erzeugen, und der zu diesem Zweck mit einem Auslösegenerator (GD) verbunden ist, der es ermöglicht, das in der Röhre (T) eingeschlossene Gas (GZ) zu ionisieren, damit die in dem durch den Generator (G) aufgeladenen Kondensatornetzwerk vorhandene Spannung einen Impuls zwischen Anode und Kathode der Lampe hervorruft.

4. Desinfektionsvorrichtung nach Anspruch 1, 2 oder 3, **dadurch gekennzeichnet, dass** die lineare Verteilung der Kondensatoren des Kondensatornetzwerks in einer zylindrischen Art und Weise verteilt ist.

5. Desinfektionsvorrichtung nach einem der vorhergehenden Ansprüche, **dadurch gekennzeichnet, dass** der durch die Koaxialanordnung mit der Lampe verbundene Generator, auch der Auslösegenerator ist.

6. Desinfektionsvorrichtung nach einem der vorhergehenden Ansprüche, **dadurch gekennzeichnet, dass** die Stromflächendichte derart ist, dass die der Lampe zugeführte Energie zwischen 20 und 500 Joules liegt.

7. Desinfektionsvorrichtung nach Anspruch 6, **dadurch gekennzeichnet, dass** die optimierte Stromflächendichte derart ist, dass die der Lampe zugeführte Energie zwischen 40 und 50 Joules liegt.

8. Desinfektionsvorrichtung nach einem der vorhergehenden Ansprüche, **dadurch gekennzeichnet, dass** das Kondensatornetzwerk (CD) eine Kapazität von C=2E/V² in Abhängigkeit von der optimalen Energie E und der Spitzenspannung V des erzeugten Signals aufweist.

9. Desinfektionsvorrichtung nach einem der vorhergehenden Ansprüche, **dadurch gekennzeichnet, dass** das Gas (GZ) der Ultraviolettlampe (L) durch eine Spannung von wenigstens 500 V zwischen der Anode (AN) und der Kathode (CAT) angeregt wird, die von dem durch den Generator geladenen Kondensatornetzwerk erzeugt wird.

10. Desinfektionsvorrichtung nach einem der vorhergehenden Ansprüche, **dadurch gekennzeichnet, dass** das Kondensatornetzwerk (CD) in Kombination mit der Auslösevorrichtung Impulse mit einer Dauer von 20 bis 40 µs und einer Anstiegszeit von weniger als 3 µs erzeugen kann.

11. Desinfektionssystem nach einem der vorhergehenden Ansprüche, **dadurch gekennzeichnet, dass** das Kondensatornetzwerk (CD) eine Vielzahl von kapazitiven Komponenten umfasst, die zwischen der Versorgungsleitung (FL) und der Rückleitung (RL) innerhalb der Koaxialanordnung (EL) verschweißt sind.

12. Desinfektionssystem, umfassend mehrere parallele Desinfektionsvorrichtungen nach einem der vorhergehenden Ansprüche zur Abdeckung entsprechend großer Flächen.

13. Desinfektionssystem nach Anspruch 12, **dadurch gekennzeichnet, dass** ein gleicher Generator mit den Koaxialanordnungen mehrerer erfindungsgemäßer Vorrichtungen verbunden ist.

14. Desinfektionssystem nach Anspruch 12, **dadurch gekennzeichnet, dass** die Parallelschaltung der Vorrichtungen die Verarbeitung mehrerer Prozesse ermöglicht.

## Claims

1. Disinfection device using a pulsed light in the ultraviolet range comprising at least one disinfection chamber (CHD), a high-voltage generator (GI), an ultraviolet lamp (L) comprising an anode (AN) and a cathode (CAT) placed in a tube (T) containing a specific gas (GZ) capable of producing ultraviolet radiation by excitation of the gas (GZ), a device for triggering a pulse between anode (AN) and cathode (CAT) and a coaxial setup (EL) for feeding the ultraviolet lamp (L) arranged between the generator (GI) and the ultraviolet lamp (L), this coaxial setup (EL) comprising a feed line (FL) linked to the anode (AN) and a return line (RL) linked to the cathode (CAT), **characterized in that** an array of capacitors (CD) is distributed along the coaxial setup (EL) between the feed line (FL) and the return line (RL) over a length of at least the length of the lamp (L), this array of capacitors contributing to the generation of the pulse by production of electrical voltage between the anode (AN) and the cathode (CAT) of the lamp (L) in combination with the trigger device, for which the characteristics of the array of capacitors (CD) are optimized to enhance the surface current density supplied to the lamp (L) and thus reduce the scattering of the ultraviolet spectrum in the wavelengths outside of the wavelengths of the ultraviolet range C having germicidal properties.

2. Disinfection device according to Claim 1, **characterized in that** the array of capacitors (CD) is distributed over a length of at least the length of the lamp (L).

3. Disinfection device according to Claim 1 or 2, **characterized in that** the trigger device comprises a trigger ring (BD) arranged around the tube (T) to create therein an ionizing field and linked for that to a trigger generator (GD) making it possible to ionize the gas (GZ) present in the tube (T) for the voltage present in the array of capacitors charged by the generator (G) to provoke a pulse between anode and cathode of the lamp.

4. Disinfection device according to Claim 1, 2 or 3, **characterized in that** the distribution per unit length of the capacitors of the array of capacitors is distributed cylindrically.

5. Disinfection device according to any one of the preceding claims, **characterized in that** the generator linked to the lamp by the coaxial setup is also the trigger generator.

6. Disinfection device according to any one of the preceding claims, **characterized in that** the surface current density is such that the energy supplied to the lamp is between 20 and 500 Joules.

7. Disinfection device according to Claim 6, **characterized in that** the optimized surface current density is such that the energy supplied to the lamp is between 40 and 50 Joules.

8. Disinfection device according to any one of the preceding claims, **characterized in that** the array of capacitors (CD) has a capacitance of C = 2E/V² as a function of the optimal energy E and of the peak voltage V of the generator signal.

9. Disinfection device according to any one of the preceding claims, **characterized in that** the gas (GZ) of the ultraviolet lamp (L) is excited by a voltage of at least 500 V between the anode (AN) and the cathode (CAT), generated by the array of capacitors charged by the generator.

10. Disinfection device according to any one of the preceding claims, **characterized in that** the array of capacitors (CD) is capable of producing pulses of a duration of 20 to 40 µs with a rise time of a duration less than 3 µs in combination with the trigger device.

11. Disinfection device according to any one of the preceding claims, **characterized in that** the array of capacitors (CD) comprises a plurality of capacitive components soldered between the feed line (FL) and the return line (RL) in the coaxial setup (EL).

12. Disinfection system comprising several disinfection devices according to any one of the preceding claims in parallel to cover commensurately more extensive surfaces.

13. Disinfection system according to Claim 12, **characterized in that** one and the same generator is connected to the coaxial setups of several devices according to the invention.

14. Disinfection system according to Claim 12, **characterized in that** the parallel connection of said devices makes it possible to handle several processes.
